# EUROPEAN PATENT SPECIFICATION

(11) **EP 2 669 682 B1**
(45) Date of publication and mention of the grant of the patent: **19.04.2017**
(21) Application number: 12170207.0
(22) Date of filing: 31.05.2012
(51) Int. Cl.: G01N 33/574

(54) **Novel prognostic and predictive biomarkers (tumor markers) for human breast cancer**
Neuartige prognostische und prädiktive Biomarker (Tumormarker) für menschlichen Brustkrebs
Nouveaux biomarqueurs pronostiques et prédictifs (marqueurs tumoraux) pour le cancer du sein chez l'homme

(43) Date of publication of application: 04.12.2013
(73) Proprietor: Heinrich Heine Universität Düsseldorf, 40225 Düsseldorf (DE); Royer, Hans-Dieter, 40225 Düsseldorf (DE)
(72) Inventor: Royer, Hans-Dieter, 40225 Düsseldorf (DE); Kassack, Matthias, 53115 Bonn (DE); Hamacher, Alexandra, 53347 Alfter (DE); Royer-Pokora, Brigitte, 40225 Düsseldorf (DE)
(74) Representative: Krauss, Jan

(56) References cited:
- WO-A2-2012/045019
- JIE ZHANG ET AL: "Using Frequent Co-expression Network to Identify Gene Clusters for Breast Cancer Prognosis", BIOINFORMATICS, SYSTEMS BIOLOGY AND INTELLIGENT COMPUTING, 2009. IJCBS '09. INTERNATIONAL JOINT CONFERENCE ON, IEEE, PISCATAWAY, NJ, USA, 3 August 2009 (2009-08-03), pages 428-434, XP031530790, ISBN: 978-0-7695-3739-9
- KATHERINE J. MARTIN ET AL: "Prognostic Breast Cancer Signature Identified from 3D Culture Model Accurately Predicts Clinical Outcome across Independent Datasets", PLOS ONE, vol. 3, no. 8, 20 August 2008 (2008-08-20) , page e2994, XP055100448, ISSN: 1932-6203, DOI: 10.1371/journal.pone.0002994

## Description

The present invention relates to a novel predictive biomarker (tumor marker) for human breast cancer. This biomarker helps to make clinical decisions that result in improvements in the clinical outcome overall survival.

### Background of the invention

Breast cancer is a family of diseases that consists of major categories, including HER-2-positive breast cancer; "triple-negative" tumours that are ER-negative, progesterone receptor-negative, and HER-2-negative; and hormonally sensitive breast cancers. The estrogen receptor-expressing (ER-positive) breast cancers are the most prevalent. For the therapy of HER2-overexpressing metastatic breast cancer, platinum complexes have been used in combination with paclitaxel and trastuzumab, a humanized monoclonal IgG₁ that binds the extracellular domain of the ERBB2 (HER-2/neu) receptor. For the treatment of HER-2-positive locally advanced breast cancer, a combination of docetaxel, cisplatin, and trastuzumab can be used as primary systemic therapy. Furthermore, several ongoing phase II studies explore the use of platinum containing compounds for the therapy of breast cancer, including "triple-negative" (ER-, PgR-, and HER-2-negative) breast carcinomas. The website of clinicaltrials.gov shows all breast cancer trials which use cisplatin in various combinations with other anticancer drugs for the treatment of breast cancer. These include, for example, a combination of gemcitabine with cisplatin in the neodjuvant situation, or the treatment of metastatic breast cancer in a combination with cetuximab.

Resistance to chemotherapy is a major obstacle to the effective treatment of many tumour types. Although many anticancer therapies can alter tumour growth, in most cases the effect is not long lasting.

Resistance to chemotherapy can occur prior to drug treatment (primary or innate resistance) or may develop over time following exposure (acquired resistance). Patients with breast cancer who are, for example, treated with an anthracycline and/or a taxane commonly develop resistance to one or both of the drugs. In some patients, prolonged exposure to a single chemotherapeutic agent may lead to the development of resistance to multiple other structurally unrelated compounds, known as cross-resistance or multidrug resistance (MDR). In primary resistance, MDR can occur without prior exposure to chemotherapy.

The development of cellular resistance to anticancer drugs is a dynamic biological process of high complexity. To better understand and react to this clinically important issue, novel approaches like systems biology are needed. In these approaches, biomarkers play an important role.

WO 99/06831 describes breast cancer resistance genes, methods for their detection and uses thereof.

US 2009/203051 describes cancer markers that were developed to detect diseases characterized by increased expression of apoptosis-suppressing genes, such as aggressive cancers. Diagnosis and assessment of amplification levels of 66 genes shown to be amplified were useful in prediction of patient outcome, of patient's response and drug resistance in breast cancer. Certain genes were found to be high priority therapeutic targets by the identification of recurrent aberrations involving genome sequence, copy number and/or gene expression are associated with reduced survival duration in certain diseases and cancers, specifically breast cancer. Inhibitors for the therapy of these non-responsive cancers are proposed.

Gosepath et al. (in Gosepath EM, Weykam S, Gruenewald E, Ko Y, Wiese M, Kassack MU. Differential gene expression studies in cisplatin-sensitive versus cisplatin-resistant human cancer cell lines. Int J Clin Pharmacol Ther. 2004 Nov; 42(11):637-9) describe gene expression studies in cisplatin-sensitive versus cisplatin-resistant human cancer cell lines and related protocols.

Similarly, Ahn et al. (in Myung-Ju Ahn, M.D., Young-Do Yoo, Ki-Hwan Lee, Joon-Ik Ahn, Dong-Hyun Yu, Hye-Sook Lee, Ho-Suck Oh, Jung-Hye Choi, and Yong-Sung Lee, cDNA Microarray Analysis of Differential Gene Expression in Gastric Cancer Cells Sensitive and Resistant to 5-Fluorouracil and Cisplatin; Cancer Res Treat. 2005 February; 37(1): 54-62) describe gene expression studies in gastric cancer as one of the most prevalent cancers worldwide. 5-fluorouracil (5-FU) and cisplatin are the most commonly used drugs for the treatment of gastric cancer. However, a significant number of tumors often fail to respond to chemotherapy.

WO 2012045019 generally relates to a molecular classification of disease and particular to methods and compositions for determining BRCA deficiency (emphasis added). CCP (cell cycle progression genes), which include *ASPM*, are used to monitor the functional status of BRCA genes. The impact of individual CCP genes for breast cancer prognosis is not addressed.

Zhang et al. (Using frequent co-expression network to identify gene clusters for breast cancer prognosis, Proc Int Joint Conf Bioinforma Syst Biol Intell Comput. 2009 Aug 3:428-434) discloses potential biomarkers for breast carcinoma prognosis of ER-positive patients that were identified using co-expression network analyses (CODENSE algorithm). The data sets that were analyzed give no information on specific therapies and therefore no information regarding response to chemotherapy.

Martin et al. (Prognostic breast cancer signature from 3D culture model accurately predicts clinical outcome across independent datasets, PLoS One. 2008 Aug 20;3(8):e2994) describes gene expression profiles of differentiated breast acini, and a group of downregulated genes (n = 22) was identified. When the expression of these genes was investigated in three breast cancer datasets, it was found that increased expression in breast tumors of some of these genes was associated with bad prognosis. Two datasets were used, "Wang" and "Sorlie". *ASPM* was prognostic in the "Wang" data set. In this case, patient relapse was the endpoint. No information on therapy response was given in this paper.

Finally, Gillet et al. (in Gillet JP, Calcagno AM, Varma S, Davidson B, Bunkholt Elstrand M, Ganapathi R, Kamat A, Sood AK, Ambudkar SV, Seiden M, Rueda BR, Gottesman MM.. Multidrug Resistance-Linked Gene Signature Predicts Overall Survival of Patients With Primary Ovarian Serous Carcinoma. Clin Cancer Res. 2012 Apr 5) disclose a study that assesses the ability of multidrug resistance (MDR)-associated gene expression patterns to predict survival in patients with newly diagnosed carcinoma of the ovary. The scope of the research is described to differ substantially from that of previous reports, as a very large set of genes was evaluated whose expression has been shown to affect response to chemotherapy. For this, a customized TaqMan Low Density Array, a highly sensitive and specific assay, was applied, to study the expression profiles of 380 MDR-linked genes in 80 tumor specimens collected at initial surgery to debulk primary serous carcinoma. The RNA expression profiles of these drug resistance genes were correlated with clinical outcomes. Leave-one-out cross-validation was used to estimate the ability of MDR gene expression to predict survival. Although gene expression alone does not predict overall survival (P=0.06), four covariates (age, stage, CA125 level and surgical debulking) do (P=0.03). When gene expression was added to the covariates, an 11-gene signature was found that provides a major improvement in overall survival prediction (log-rank statistic P less than 0.003). The predictive power of this 11-gene signature was confirmed by dividing high and low risk patient groups, as defined by their clinical covariates, into four specific risk groups based on expression levels. The 11-gene signature is described to allow a more precise prognosis for patients with serous cancer of the ovary treated with carboplatin- and paclitaxel-based therapy. These 11 new targets are described to offer opportunities for new therapies to improve clinical outcome in ovarian cancer.

Despite the above approaches, there is a continued need to provide new biomarkers for predicting the future behavior of an established breast cancer, either in the absence or after application of therapy (local or systemic). Predictive biomarkers predict response or resistance to a specific therapy. Further objects and advantages will become apparent to the person of skill when reading the following more detailed description of the present invention.

In a preferred first aspect of the present invention, the invention thus relates to a method for predicting response or resistance to specific therapies (overall survival, OS) in a breast cancer patient that is undergoing cancer treatment, comprising determining the expression level of the biomarker ASPM in a biological sample obtained from said cancer patient, wherein an increase or higher level of expression compared to the median thereof in a given cancer patient population is predictive for an adverse response or resistance outcome for said patient.

Predictive biomarkers are associated with overall survival (OS) and prognostic biomarkers with disease free survival (DFS) in said breast cancer patient.

**Table 1: Tumor markers (biomarkers) as disclosed. Significance (p-values) for overall survival and disease free survival**

| **Survival data:** | | |
|---|---|---|
| **chemotherapy resistance** | | |
| **genes** | **p value OS** | **p value DFS** |
| KM plotter breast cancer | | |
| (http://kmplot.com/analysis) | overall survival | disease free survival |
| ABCC5 | 0.0164 | NA |
| ABHD5 | 0.0229 | NA |
| ACLY | 0.0524 | NA |
| ACOT7 | 0.0082 | p ZERO |
| ALG8 | 0.0237 | NA |
| AP1G1 | 0.0003 | p ZERO |
| APEX2 | 0.0004 | NA |
| ARG2 | 0.0027 | NA |
| ASPM | p ZERO | p ZERO |
| ATAD2 | 0.0459 | p ZERO |
| ATP9A | 0.0185 | 0.0322 |
| BACH1 | 0.0975 | 0.0001 |
| BLM | 0.041 | NA |
| BOP1 | 0.0331 | 0.0002 |
| BRCA1 | 0.0021 | p ZERO |
| CCNE1 | 0.0003 | p ZERO |
| CCNE2 | 0.0002 | p ZERO |
| CCNF | 0.0015 | 0.0075 |
| CCT5 | 0.0061 | p ZERO |
| CD2AP | NA | p ZERO |
| CDC25A | NA | p ZERO |
| CDC45L | 0.0059 | p ZERO |
| CDC7 | 0.0002 | 0.01 |
| CDK2 | 0.03 | NA |
| CENPA | 0.0107 | p ZERO |
| CENPE | 0.0026 | p ZERO |
| CENPF | p ZERO | p ZERO |
| CEP55 | 0.0144 | p ZERO |
| CFDP1 | 0.0022 | p ZERO |
| CHAC1 | 0.0151 | 0.0319 |
| CHD2 | 0.0413 | NA |
| CLN6 | 0.0213 | NA |
| CNOT6 | 0.0245 | p ZERO |
| COL13A1 | 0.0117 | 0.0004 |
| CPOX | 0.0053 | NA |
| CTNNG | 0.001 | NA |
| CTPS | 0.0013 | NA |
| CTSA | 0.0205 | NA |
| CTSL1 | 0.0619 | p ZERO |
| DDEF2 | 0.0002 | p ZERO |
| DDX46 | 0.0161 | 0.0213 |
| DHFR | 0.0118 | p ZERO |
| DONSON | 0.0002 | p ZERO |
| DTL | 0.0001 | p ZERO |
| DYNC1H1 | NA | 0.0591 |
| E2F1 | 0.0005 | p ZERO |
| E2F8 | 0.0225 | p ZERO |
| ECT2 | 0.0214 | p ZERO |
| EML4 | 0.0341 | NA |
| ESPL1 | p ZERO | 0.0132 |
| EZH2 | NA | p ZERO |
| FA2H | 0.0666 | NA |
| FAM46A | 0.0196 | NA |
| FANCI | 0.0196 | p ZERO |
| FN1 | 0.0767 | NA |
| FREQ | 0.1226 | 0.0031 |
| GALNT14 | 0.0277 | 0.0052 |
| GARS | 0.0637 | p ZERO |
| GCLM | 0.0017 | p ZERO |
| GPNMB | 0.089 | p ZERO |
| GTSE1 | p ZERO | p ZERO |
| HIP1R | 0.0174 | NA |
| HMGCR | 0.0044 | p ZERO |
| HMGCS1 | NA | 0.0012 |
| HN1 | NA | p ZERO |
| HPRT1 | 0.0085 | p ZERO |
| HSPC171 | 0.0128 | p ZERO |
| HYPK | 0.0343 | p ZERO |
| IFIT1 | 0.0079 | 0.0001 |
| IFIT3 | 0.0082 | 0.0275 |
| KIF11 | 0.0012 | p ZERO |
| KRT19 | 0.0505 | 0.0182 |
| LARP4 | 0.0169 | 0.0002 |
| LCN2 | 0.0033 | NA |
| LMNB1 | 0.0017 | p ZERO |
| LSR | 0.0115 | p ZERO |
| MCM10 | 0.0086 | p ZERO |
| MCM3 | 0.0045 | 0.0365 |
| MCM6 | 0.008 | p ZERO |
| MCM7 | 0.0014 | p ZERO |
| MLF1IP | 0.0002 | p ZERO |
| MMP1 | 0.0021 | p ZERO |
| MORC2 | 0.0145 | p ZERO |
| MSH6 | 0.0006 | p ZERO |
| MT1X | 0.0324 | 0.0094 |
| NCAPG | 0.0002 | p ZERO |
| NUDT4 | NA | 0.0029 |
| NUP107 | 0.0178 | p ZERO |
| OGFOD1 | 0.0064 | NA |
| P2RY2 | 0.0155 | NA |
| PCNA | 0.0127 | p ZERO |
| PDCD6IP | 0.0219 | NA |
| PDSS1 | 0.0114 | 0.001 |
| PGK1 | p ZERO | p ZERO |
| PHGDH | 0.0304 | 0.0047 |
| PKMYT1 | 0.0002 | NA |
| POLQ | 0.0031 | Na |
| PPFIA4 | 0.0663 | NA |
| PRC1 | 0.0001 | p ZERO |
| PSMD12 | 0.0105 | p ZERO |
| PUS1 | 0.0249 | p ZERO |
| RAB3D | 0.0971 | NA |
| RACGAP1 | p ZERO | p ZERO |
| RBM14 | 0.0225 | NA |
| RPGRIP1L | 0.001 | 0.0361 |
| RRM1 | 0.061 | p ZERO |
| RRM2 | 0.0002 | p ZERO |
| RUVBL1 | NA | p ZERO |
| SETD8 | 0.0001 | 0.0006 |
| SEZ6L2 | 0.0017 | NA |
| SLC25A13 | 0.0311 | p ZERO |
| SOD1 | 0.0716 | p ZERO |
| SPC25 | 0.0025 | p ZERO |
| SQLE | 0.0026 | p ZERO |
| SRPK1 | 0.0023 | p ZERO |
| STC1 | 0.0038 | NA |
| TFB2M | 0.042 | p ZERO |
| THOP1 | 0.0031 | 0.0324 |
| TK1 | p ZERO | p ZERO |
| TMEM132A | 0.0078 | 0.0008 |
| TMEM16A | 0.0536 | 0.0758 |
| TRIP 13 | 0.0002 | p ZERO |
| TSEN2 | 0.0533 | NA |
| TSPAN13 | 0.0364 | p ZERO |
| TTK | 0.0416 | p ZERO |
| TUBA1B | 0.0338 | 0.0681 |
| TUBB | NA | p ZERO |
| TUBB2C | 0.0089 | p ZERO |
| TUBB3 | 0.0034 | p ZERO |
| TXNRD1 | 0.0061 | p ZERO |
| UCHL5 | NA | p ZERO |
| USP32 | 0.0848 | 0.002 |
| WDR26 | NA | p ZERO |
| ZWINT | 0.0011 | p ZERO |

NA: no significant association with disease free or overall survival (p >0.05)

Disclosed is a set of at least one, preferably 2, 3, 4, 5, or 6 markers selected from ASPM, CENPF, GTSE1, PGK1, RACGAP1, and TK1.

Disclosed is a method for identifying an anti-cancer agent, comprising the steps of providing at least one biomarker or a panel of biomarkers as disclosed in a biological sample from a patient, contacting said sample with at least one compound that potentially modifies the expression and/or biological activity of said at least one biomarker, and identifying a compound that modifies the expression and/or biological activity of said at least one resistance biomarker.

Also disclosed is a method of treating and/or preventing breast cancer, in particular chemotherapy-resistant cancer in a patient in need of said treatment, comprising performing a method according to the present invention, and providing a suitable chemotherapeutic anti-cancer treatment to said patient, wherein said treatment is based, at least in part, on the results of the method according to the present invention.

Disclosed is a diagnostic kit comprising materials for performing a method according to the present invention in one or separate containers, preferably comprising at least one biomarker-specific antibody, optionally together with auxiliary agents and/or instructions for performing said method according to the present invention.

The invention is based on the new and successful attempts of the inventors to identify prognostic and/or predictive biomarkers for breast cancer. For this, based on mimicking the situation in the clinics, the inventors generated chemotherapy resistant breast cancer cell lines through a weekly treatment of the cells with the respective drug for six months. This approach was based on clinical data of human breast cancer which show that treatment resistance in a breast cancer patient develops in a time frame ranging from months to years.

Of note, this approach differs from the common approaches in the field, which analyze samples from cancer patients (as much as they are available) in order to identify markers. All these samples are derived from a more or less inhomogeneous background of treatments and disease histories, and thus are prone to bias and the selective over-identification (and thus over-representation) of biomarkers. The present approach identified markers following a standard treatment scheme as applied by the attending physician, and thus provides unbiased and objective markers.

Then, based on these cell lines as generated in view of the clinically relevant treatment regimen, using dual color microarrays (Agilent), resistance associated genes (gene signatures or panels) were generated. Using the "KM plotter" (http://kmplot.com/analysis) in the context of breast cancer, the resistance-associated genes were analyzed and those related to overall survival (OS) and disease free survival (DFS) were identified. Of note, by using this approach, these biomarkers have already been validated using collective patient data, but can also be applied to individual patients and/or patient-subgroups (such as, for example, described below).

Prognosis for patients with established primary or metastatic breast cancer is defined as the prediction of future behavior of an established tumor, either in the absence of or after application of therapy (local or systemic). Prognostic factors can be divided into two categories: 1) those that predict relapse or progression independent of future treatment effects (herein designated as "prognostic" factors) and 2) those that predict response or resistance to a specific therapy (herein designated as "predictive" factors) (see also Hayes et al. 1996. Journal of the National Cancer Institute 88, 1456-1466). The knowledge of biomarker data will lead to a clinical decision that results in a more favorable clinical outcome. Biomarkers help to reliably make clinical decisions that result in improvements in one of four clinical outcomes: overall survival, disease free survival, quality of life, or cost of care.

Disease free survival (DFS)-associated biomarkers are prognostic biomarkers, that predict relapse or progression independent of future treatment effects. These factors (biomarkers) serve to identify patients at risk (risk group identification). Usually, patients at risk have a more aggressive tumour. Using the risk as established, the attending physician can decide, whether a certain therapy, in particular chemotherapy, shall be applied or not.

Overall survival (OS)-associated biomarkers are predictive biomarkers, that predict response or resistance to a specific therapy (or combinations of therapies). These markers serve to decide, which therapy, in particular chemotherapy, shall be applied or not. Ideally, a panel of predictive biomarkers shall be used in order to decide on an individualized and/or personalized therapy, in particular which therapy scheme shall be followed, and which drugs shall be used.

Disclosed are biomarkers selected from the group consisting of ABCC5, ABHD5, ACLY, ACOT7, ALG8, AP1G1, APEX2, ARG2, ASPM, ATAD2, ATP9A, BACH1, BLM, BOP1, BRCA1, CCNE1, CCNE2, CCNF, CCT5, CD2AP, CDC25A, CDC45L, CDC7, CDK2, CENPA, CENPE, CENPF, CEP55, CFDP1, CHAC1, CHD2, CLN6, CNOT6, COL13A1, CPOX, CTNNG, CTPS, CTSA, CTSL1, DDEF2, DDX46, DHFR, DONSON, DTL, DYNC1H1, E2F1, E2F8, ECT2, EML4, ESPL1, EZH2, FA2H, FAM46A, FANCI, FN1, FREQ, GALNT14, GARS, GCLM, GPNMB, GTSE1, HIP1R, HMGCR, HMGCS1, HN1, HPRT1, HSPC171, HYPK, IFIT1, IFIT3, KIF11, KRT19, LARP4, LCN2, LMNB1, LSR, MCM10, MCM3, MCM6, MCM7, MLF1IP, MMP1, MORC2, MSH6, MT1X, NCAPG, NUDT4, NUP107, OGFOD1, P2RY2, PCNA, PDCD6IP, PDSS1, PGK1, PHGDH, PKMYT1, POLQ, PPFIA4, PRC1, PSMD12, PUS1, RAB3D, RACGAP1, RBM14, RPGRIP1L, RRM1, RRM2, RUVBL1, SETD8, SEZ6L2, SLC25A13, SOD1, SPC25, SQLE, SRPK1, STC1, TFB2M, THOP1, TK1, TMEM132A, TMEM16A, TRIP 13, TSEN2, TSPAN13, TTK, TUBA1B, TUBB, TUBB2C, TUBB3, TXNRD1, UCHL5, USP32, WDR26, and ZWINT for predicting relapse or progression independent of future treatment effects (disease free survival, DFS) and predicting response or resistance to specific therapies (overall survival, OS) in said breast cancer patient. These biomarkers help to make clinical decisions that result in improvements in one of four clinical outcomes: overall survival, disease free survival, quality of life, or cost of care. In other words, these biomarkers predict success of a therapy in a breast cancer patient.

In the context of the present disclosure, a "success of a therapy in a breast cancer patient" (or group of patients) shall mean any therapy that reduces, eradicates, and/or slows down the growth of cancer cells in a breast cancer patient. The cancer cells to be treated can further include metastasized cells, i.e. tumor cells outside of the breast tissues based on the initial breast cancer.

"Disease free survival" shall mean the length of time for a specific disease during which a patient survives with no sign of the disease. Disease-free survival may be used in a clinical study or trial to help to identify patients at risk (risk group identification), and to subsequently decide which treatment the patient should receive.

"Overall survival" or "overall survival rate" shall mean the percentage of people in a study or treatment group who are alive for a certain period of time after they were diagnosed with and treated for cancer. The overall survival rate is often stated as a time-related survival, such as, for example, five-year survival rate, which is the percentage of people in a study or treatment group who are alive five years after diagnosis or treatment. Overall survival (OS) is the gold standard primary end point to evaluate the outcome of any drug, biologic intervention, or procedure that is assessed in oncologic clinical trials. OS is recognized as being unambiguous, unbiased, with a defined end point of very high clinical relevance, and positive results provide most significant evidence that a given treatment extends the life of a patient (Driscoll JJ, Rixe O. Overall survival: still the gold standard: why overall survival remains the definitive end point in cancer clinical trials. Cancer J. 2009 Sep-Oct;15(5):401-5.)

A "biomarker" or "marker" in the context of the present invention shall generally mean a biological molecule found in blood, other body fluids, or tissues that is a sign of a normal or abnormal process, or of a condition or disease,"(NCI) such as cancer. Biomarkers typically differentiate an affected patient from a person without the disease. The alterations can be due

In the experiments that form the basis for the present invention, a number of markers have been identified as being related to the prognosis and/or overall survival of breast cancer patients. Many of these markers have not been known to be linked to clinical outcome of a breast cancer patient. Furthermore, a use as prognostic marker e.g. disease free survival (DFS) and/or predictive marker for overall survival (OS) has also not been described.

For the purposes of the present invention, in one preferred embodiment, markers that yet have not been linked with breast cancer, and/or disease free survival (DFS) and/or of overall survival (OS) are analyzed together (e.g. as part of a panel of markers comprising 2, 3, 4, 5 or more markers).

The marker ABCC5 has been described as a predictor of the pathologic response to neoadjuvant chemotherapy poor survival BRCA, one report 55 patients (Park et al., Breast Cancer Research and Treatment (2006) 99: 9-17).

The marker ALG8 has been described in one report as amplified in breast cancer, and to be linked with a bad prognosis.

The marker ATAD2 has been described to be linked with a poor prognosis in breast cancer.

The marker to be used according to the present invention, ASPM, has been described to be involed in glioblastoma, the centrosome function, and to be down-regulated by BRCA1 siRNAs.

The marker BRCA1 has been extensively described to cause a familiar form or inherited breast cancer, and was associated with overall survival (Margeli et al., PLoS ONE 5(3): e9499. doi:10.1371/journal.pone.0009499).

The markers CCNE1 and CCNE2 have been described in several reports to be related with outcome. CCNE1 is related with outcome in systemically untreated patients and trastuzumab resistance to therapy in tamoxifen treated patients. CCNE2 is described as a prognostic marker for lymph node negative breast cancer patients, and to be prognostic in nodal-negative breast cancer (Clin Cancer Res 2006;12:3319-3328).

In the experiments that form the basis for the present invention, a number of markers have been identified as being related to the prognosis and/or overall survival of breast cancer patients. Many of these markers have not been known to be linked to clinical outcome of a breast cancer patient. Furthermore, a use as prognostic marker e.g. disease free survival (DFS) and/or predictive marker for overall survival (OS) has also not been described.

For the purposes of the present invention, in one preferred embodiment, markers that yet have not been linked with breast cancer, and/or disease free survival (DFS) and/or of overall survival (OS) are analyzed together (e.g. as part of a panel of markers comprising 2, 3, 4, 5 or more markers).

The marker ABCC5 has been described as a predictor of the pathologic response to neoadjuvant chemotherapy poor survival BRCA, one report 55 patients (Park et al., Breast Cancer Research and Treatment (2006) 99: 9-17).

The marker ALG8 has been described in one report as amplified in breast cancer, and to be linked with a bad prognosis.

The marker ATAD2 has been described to be linked with a poor prognosis in breast cancer.

The marker ASPM has been described to be involed in glioblastoma, the centrosome function, and to be down-regulated by BRCA1 siRNAs.

The marker BRCA1 has been extensively described to cause a familiar form or inherited breast cancer, and was associated with overall survival (Margeli et al., PLoS ONE 5(3): e9499. doi:10.1371/journal.pone.0009499).

The markers CCNE1 and CCNE2 have been described in several reports to be related with outcome. CCNE1 is related with outcome in systemically untreated patients and trastuzumab resistance to therapy in tamoxifen treated patients. CCNE2 is described as a prognostic marker for lymph node negative breast cancer patients, and to be prognostic in nodal-negative breast cancer (Clin Cancer Res 2006;12:3319-3328).

The marker CCT5 has been described in the context of a prediction of metastatic relapse in node-positive breast cancer (Campone et al., Breast Cancer Res Treat (2008) 109:491-501).

The marker CDC7 has been described as being overexpressed in p53 mutant aggressive breast cancers (Rodriguez-Acebes et al., The American Journal of Pathology, Vol. 177, No. 4, October 2010).

The marker CDC25A has been described as linked to poor survival.

The marker CDK2 has been described as a prognostic indicator for early breast cancer (Kim et al., Annals of Oncology 19: 68-72, 2008).

The marker CEACAM6 has been described to predict recurrence after tamoxifen treatment.

The marker CENPE in one report has been described to correlate with CCNB1, which is associated with poor prognosis.

The marker CENPF has been described to be predictive of metastatic relapse in node-positive breast cancer (Campone et al., Breast Cancer Res Treat (2008) 109:491-501) but only as part of several genes predicting metastatic relapse.

The marker CEP55 has been described to be part of a prognostic signature in estrogen receptor positive breast cancer, derived from a 3D cell culture model (Martin et al PLoS ONE 3(8): e2994. doi:10.1371/journal.pone.0002994).

The marker CHAC1 has recently been described to maybe be an independent indicator for elevated risk of cancer recurrence in breast and ovarian cancer.

The marker CTSL1 has been described as being of prognostic and predictive value in operable breast cancer patients (Cathepsins D and L) (Jagodic et al., Neoplasma (2005); 52 (1):1-9).

The marker E2F1 has been described as part of a two gene signature in breast cancer survival, and reduced DFS.

The marker EZH2 has been described as a marker for bad clinical outcome and poor prognosis in inflammatory breast cancer (Gong et al., Cancer 2011;00:000-000. DOI: 10.1002/cncr.26179).

The marker FN1 has been described in the prognosis and endocrine therapy response of breast cancer (Helleman et al., Clin Cancer Res 2008;14:5555-5564).

The marker GPNMB has been described as prognostic indicator of recurrence.

The marker GTSE1 is up-regulated in lung cancer tissues compared to the adjacent normal tissues, especially in adenocarcinoma and squamous cell carcinoma. No statistically significant correlation, however, was observed between GTSE1, the clinical features and survival (Tian T, Zhang E, Fei F, Li X, Guo X, Liu B, Li J, Chen Z, Xing J. Up-regulation of GTSE1 lacks a relationship with clinical data in lung cancer. Asian Pac J Cancer Prev. 2011;12(8):2039-43).

The marker IFIT1 has been described as part of the IFN-related DNA damage resistance signature (IRDS) predicting some resistance in breast cancer.

The marker IGFBP3 has been described to be associated with poor clinical outcome (Kim et al., Oncology Reports 23: 989-995, 2010).

The marker KRT19 has been described as part of an mRNA panel consisting of TWIST 1, CK19, and hMAM in operable breast cancer patients' disseminated cells, and to be prognostic.

The marker LCN2 has been described to be linked with poor prognosis in primary breast cancer (Bauer et al., Breast Cancer Res Treat (2008) 108:389-397).

The marker MMP1 has been described as a metastatic factor related with poor outcome, but without real clinical data.

In one report, the marker NRP2 has been described to may be prognostic.

The marker PCNA has been described to be prognostic, and to be a proliferation marker.

The marker PDCD6IP has been described to be located in a three gene amplicon associated with recurrence (i.e. indirect evidence) of cancer.

The marker PGK1 has been described as part of panels in the analysis of breast cancer chemotherapy (Cortesi L, Barchetti A, De Matteis E, Rossi E, Della Casa L, Marcheselli L, Tazzioli G, Lazzaretti MG, Ficarra G, Federico M, Iannone A. Identification of protein clusters predictive of response to chemotherapy in breast cancer patients. J Proteome Res. 2009 Nov;8(11):4916-33).

The marker PKP3 has been described to be associated with node positivity and grade of cancer, and thus the tumor biology.

The marker PLAUR has been described to be prognostic in breast cancer, but to be clinically irrelevant in ovarian cancer (Kotzschl et al Biological Chemistry, SSN (online) 1437-4315 DOI: 10.1515/BC.2011.166).

In one report, the marker POLQ has been described to be related with poor prognosis.

In one report, the marker RAB3D has been described to be related with poor prognosis.

The marker RACGAP1 has been described to be maybe involved in carcinogenesis (Katoh M, Katoh M. Characterization of human ARHGAP10 gene in silico. Int J Oncol. 2004 Oct;25(4):1201-6).

The marker RRM2 has been described as part of a prognostic signature derived from a 3D cell culture model (Martin et al PLoS ONE 3(8): e2994. doi:10.1371/journal.pone.0002994).

The marker SQLE has been described to indicate poor clinical outcome (Helms et al British Journal of Cancer (2008) 99, 774 - 780).

The marker TK1 has been described potential marker for a prognosis (He et al., Nucleosides, Nucleotides and Nucleic Acids, 29:352-358, 2010).

In one report, the marker TUBB3 has been described to be linked with bad prognosis, but to be non-predictive.

In one report, the marker TXNRD1 has been described to be prognostic.

For the markers ABHD5, ACLY, ACOT7, AP1G1, APEX2, ARG2, ASPM, ATP9A, BACH1, BLM, BOP1, CCNF, CD2AP, CDC45L, CENPA, CFDP1, CHD2, CLN6, CNOT6, COL13A1, CPOX, CTNNG, CTPS, CTSA, DDEF2, DDX46, DHFR, DONSON, DTL, DYNC1H1, E2F8, ECT2, EML4, ESPL1, FA2H, FAM46A, FANCI, FREQ, GALNT14, GARS, GCLM, GTSE1, HIP1R, HMGCR, HMGCS1, HN1, HPRT1, HSPC171, HYPK, IFIT3, CTNNG, KIF11, LARP4, LMNB1, LSR, MCM10, MCM3, MCM6, MCM7, MLF1IP, MORC2, MSH6, MT1X, NCAPG, NUDT4, NUP107, OGFOD1, P2RY2, PDSS1, PGK1, PHGDH, PKMYT1, PPFIA4, PRC1, PSMD12, PUS1, RACGAP1, RBM14, RPGRIP1L, RRM1, RUVBL1, SETD8, SEZ6L2, SLC25A13, SOD1, SPC25, SRPK1, STC1, TFB2M, THOP1, TMEM132A, TMEM16A, TRIP13, TSEN2, TSPAN13, TTK, TUBA1B, TUBB, TUBB2C, UCHL5, USP32, WDR26, and ZWINT, no clinical data with respect to breast cancer has been published.

In another preferred embodiment of the present invention, the biomarkers according to the present invention can be analyzed and thus a diagnosis can be established based on different subsets of breast cancer biomarkers. Thus, in one preferred embodiment of the present invention, said breast cancer is a luminal A breast cancer, and at least one marker is selected from the group consisting of ABCC5, ACLY, ASPM, ATAD2, ATP9A, BLM, BOP1, BRCA1, CCNE1, CCNE2, CCNF, CCT5, CDC25A, CDC45L, CDC7, CENPA, CENPE, CENPF, CEP55, CFDP1, CLN6, COL13A1, CTNNG, CTPS, CTSA, CTSL1, DHFR, DONSON, DTL, E2F1, E2F8, ECT2, EML4, EPN3, ESPL1, EZH2, FAM46A, FANCI, FREQ, GALNT14, GARS, GCLM, GPNMB, GPR157, GTSE1, HMGCR, HMGCS1, HN1, HPRT1, KIF11, KRT19, LMNB1, MCM10, MCM3, MCM6, MLF1IP, MORC2, MSH6, NCAPG, NEIL3, NFKBIL2, NUDT4, NUP107, P2RY2, PCNA, PDSS1, PKMYT1, PRC1, PSMD12, PUS1, RACGAP1, RRM2, SETD8, SPC25, SOD1, SQLE, SRPK1, TK1, TMEM132A, TMEM16A, TRIP13, TTK, TUBA1B, TUBB, TXNRD1, UCHL5, and ZWINT.

It was found in the experiments of the present invention that several of the biomarkers according to the present invention can be used individually for predicting response or resistance to specific therapies (overall survival, OS). Predictive biomarkers are associated with overall survival (OS) in said breast cancer patient. It was also found in the experiments of the present invention that several of the markers were statistically closely related with the clinical endpoint overall survival (OS), as expressed by their respective p-value. The following tables show the biomarkers and their respective p-values, wherein a value closer to zero indicates a stronger link with the parameters DFS and/or OS and thus a better suitability to establish a respective diagnosis. All values have been calculated using the KM plotter as above.

**Table 2: Markers related with luminal A breast cancer. The symbols "+", "-", "+/-", and "++" indicate the suitability of the markers for the determinations.**

| | | | | **preferred** | **preferred** |
|---|---|---|---|---|---|
| **Marker** | **p-value OS** | **p-value DFS** | **preferred OS** | **DFS** | **OS/DFS** |
| ABCC5 | 0.0007 | NA | + | - | +/- |
| ACLY | 0.0027 | NA | + | - | +/- |
| ASPM | 0.04 | p ZERO | + | + | ++ |
| ATAD2 | 0.05 | p ZERO | + | + | ++ |
| ATP9A | 0.0076 | 0.0033 | + | + | ++ |
| BLM | 0.01 | NA | + | - | +/- |
| BOP1 | NA | 0.0097 | - | + | -/+ |
| BRCA1 | 0.0019 | p ZERO | + | + | ++ |
| CCNE1 | 0.0016 | NA | + | - | +/- |
| CCNE2 | 0.0177 | p ZERO | + | + | ++ |
| CCNF | 0.0025 | 0.0049 | + | + | ++ |
| CCT5 | 0.01 | p ZERO | + | + | ++ |
| CDC25A | 0.0477 | p ZERO | + | + | ++ |
| CDC45L | 0.0144 | p ZERO | + | + | ++ |
| CDC7 | 0.0086 | 0.0105 | + | + | ++ |
| CENPA | 0.0057 | p ZERO | + | + | ++ |
| CENPE | NA | p ZERO | - | + | -/+ |
| CENPF | 0.0559 | 0.0217 | + | + | ++ |
| CEP55 | 0.0034 | p ZERO | + | + | ++ |
| CFDP1 | NA | P ZERO | - | + | -/+ |
| CLN6 | 0.0134 | NA | + | - | +/- |
| COL13A1 | NA | 0.0005 | - | + | -/+ |
| CTNNG | 0.0159 | NA | + | - | +/- |
| CTPS | NA | 0.0162 | - | + | -/+ |
| CTSA | 0.0186 | 0.055 | + | + | ++ |
| CTSL1 | NA | p ZERO | - | + | -/+ |
| DHFR | NA | 0.0009 | - | + | -/+ |
| DONSON | 0.0009 | p ZERO | + | + | ++ |
| DTL | 0.0186 | P ZERO | + | + | ++ |
| E2F1 | 0.001 | 0.0004 | + | + | ++ |
| E2F8 | 0.0025 | p ZERO | + | + | ++ |
| ECT2 | 0.0172 | p ZERO | + | + | ++ |
| EML4 | 0.0086 | NA | + | - | +/- |
| EPN3 | 0.0252 | 0.0003 | + | + | ++ |
| ESPL1 | 0.007 | 0.0024 | + | + | ++ |
| EZH2 | 0.0118 | p ZER O | + | + | ++ |
| FAM46A | 0.02 | NA | + | - | +/- |
| FANCI | NA | p ZERO | - | + | -/+ |
| FREQ | 0.0371 | NA | + | - | +/- |
| GALNT14 | 0.004 | NA | + | - | +/- |
| GARS | 0.0096 | 0.0038 | + | + | ++ |
| GCLM | 0.0021 | p ZERO | + | + | ++ |
| GPNMB | 0.0063 | 0.0031 | + | + | ++ |
| GPR157 | 0.003 | NA | + | - | +/- |
| GTSE1 | 0.0043 | 0.0002 | + | + | ++ |
| HMGCR | NA | 0.0046 | - | + | -/+ |
| HMGCS1 | 0.0054 | 0.0503 | + | + | ++ |
| HN1 | 0.0457 | p ZERO | + | + | ++ |
| HPRT1 | NA | P ZERO | - | + | -/+ |
| KIF11 | 0.0363 | p ZERO | + | + | ++ |
| KRT19 | NA | 0.0174 | - | + | -/+ |
| LMNB1 | 0.021 | p ZERO | + | + | ++ |
| MCM10 | 0.0109 | p ZERO | + | + | ++ |
| MCM3 | 0.0197 | NA | + | - | +/- |
| MCM6 | NA | P ZERO | - | + | -/+ |
| MLF1IP | 0.0265 | p ZERO | + | + | ++ |
| MORC2 | NA | 0.0192 | - | + | -/+ |
| MSH6 | 0.0127 | p ZERO | + | + | ++ |
| NCAPG | NA | P ZERO | - | + | -/+ |
| NEIL3 | 0.0023 | NA | + | - | +/- |
| NFKBIL2 | 0.0031 | 0.0545 | + | + | ++ |
| NUDT4 | 0.0227 | NA | + | - | +/- |
| NUP107 | 0.0007 | p ZERO | + | + | ++ |
| P2RY2 | 0.0139 | NA | + | - | +/- |
| PCNA | 0.0118 | p ZERO | + | + | ++ |
| PDSS1 | 0.0183 | NA | + | - | +/- |
| PKMYT1 | p ZERO | NA | + | - | +/- |
| PRC1 | 0.0462 | p ZERO | + | + | ++ |
| PSMD12 | 0.0169 | P ZERO | + | + | ++ |
| PUS1 | NA | 0.0141 | - | + | -/+ |
| RACGAP1 | 0.0254 | p ZERO | + | + | ++ |
| RRM2 | 0.0057 | p ZERO | + | + | ++ |
| SETD8 | 0.0097 | NA | + | - | +/- |
| SOD1 | NA | p ZERO | - | + | -/+ |
| SPC25 | 0.0178 | p ZERO | + | + | ++ |
| SQLE | 0.0001 | p ZERO | + | + | ++ |
| SRPK1 | NA | P ZERO | - | + | -/+ |
| TK1 | 0.0044 | NA | + | - | +/- |
| TMEM132A | 0.0187 | 0.0042 | + | + | ++ |
| TMEM16A | NA | 0.0543 | - | + | -/+ |
| TRIP 13 | 0.0011 | p ZERO | + | + | ++ |
| TTK | 0.0219 | p ZERO | + | + | ++ |
| TUBA1B | 0.0168 | NA | + | - | +/- |
| TUBB | NA | 0.0008 | - | + | -/+ |

**NA**, no significant association with disease free or overall survival (p >0.05)

**Table 3: Markers related with luminal B breast cancer. The symbols "+", "-", "+/-", and "++" indicate the suitability of the markers for the determinations.**

| | | | **preferred** | **preferred** | **preferred** |
|---|---|---|---|---|---|
| | **OS** | **DFS** | **OS** | **DFS** | **OS/DFS** |
| ACOT7 | 0.0081 | 0.001 | + | + | ++ |
| AP2A2 | 0.04 | NA | + | - | +/- |
| CDK2 | 0.046 | NA | + | - | +/- |
| CFDP1 | NA | P ZERO | - | + | -/+ |
| CNOT6 | 0.016 | NA | + | - | +/- |
| CTNNG | 0.0198 | NA | + | - | +/- |
| CSTF3 | 0.0006 | NA | + | - | +/- |
| DDEF2 | 0.009 | 0.0049 | + | + | ++ |
| DDX46 | 0.03 | 0.0102 | + | + | ++ |
| DHFR | NA | P ZERO | - | + | -/+ |
| DTL | NA | P ZERO | - | + | -/+ |
| EAF1 | 0.02 | NA | + | - | +/- |
| GAA | 0.010 | NA | + | - | +/- |
| GDI1 | 0.0043 | NA | + | - | +/- |
| HIP1R | 0.009 | NA | + | - | +/- |
| HPRT1 | NA | 0.0001 | - | + | -/+ |
| IFIT1 | 0.0052 | NA | + | - | +/- |
| IRX5 | 0.019 | NA | + | - | +/- |
| KRT18 | 0.03 | 0.0062 | + | + | ++ |
| KRT19 | NA | 0.0006 | - | + | -/+ |
| LSR | 0.0024 | NA | + | - | +/- |
| MORC2 | NA | 0.0248 | - | + | -/+ |
| NCAPG | NA | 0.0033 | - | + | -/+ |
| NSUN6 | 0.03 | NA | + | - | +/- |
| PDCD6IP | 0.019 | 0.0539 | + | + | ++ |
| PGK1 | NA | p ZERO | - | + | -/+ |
| PKP3 | 0.02 | NA | + | - | +/- |
| PPFIA4 | 0.014 | NA | + | - | +/- |
| PSMD12 | NA | 0.0004 | - | + | -/+ |
| RACGAP1 | 0.015 | p ZERO | + | + | ++ |
| RBM14 | 0.0063 | NA | + | - | +/- |
| RUVBL1 | 0.051 | p ZERO | + | + | ++ |
| SEZ6L2 | 0.019 | NA | + | - | +/- |
| SOD1 | 0.0018 | p ZERO | + | + | ++ |
| SRPK1 | NA | 0.0166 | - | + | -/+ |
| STC1 | 0.02 | NA | + | - | +/- |
| TFB2M | 0.017 | p ZERO | + | + | ++ |
| TSPAN13 | 0.008 | 0.0004 | + | + | ++ |
| USP32 | 0.04 | NA | + | - | +/- |
| WDR26 | 0.0006 | NA | + | - | +/- |
| ZC3H12A | 0.01 | NA | + | - | +/- |

**NA**, no significant association with overall or disease free survival (p >0.05)

**Table 4: Markers specific for basal breast cancer (similar to triple negative breast cancer, TNBC). The symbols "+", "-", "+/-", and "++" indicate the suitability of the markers for the determinations.**

| | | | **preferred** | **preferred** | **preferred** |
|---|---|---|---|---|---|
| | **OS** | **DFS** | **OS** | **DFS** | **OS/DFS** |
| ABHD5 | 0.0001 | 0.0061 | + | + | ++ |
| CD2AP | 0.0278 | p ZERO | + | + | ++ |
| DMBT1 | 0.0551 | NA | + | - | +/- |
| DTL | NA | 0.0012 | - | + | -/+ |
| HIP1R | 0.0052 | NA | + | - | +/- |
| HPRT1 | NA | 0.0487 | - | + | -/+ |
| HYPK | 0.041 | p ZERO | + | + | ++ |
| LCN2 | 0.07 | NA | + | - | +/- |
| MMP1 | 0.01 | NA | + | - | +/- |
| MT1X | 0.062 | NA | + | - | +/- |
| NCAPG | NA | 0.0028 | - | + | -/+ |
| PDCD6IP | 0.04 | NA | + | - | +/- |
| SLC25A13 | 0.057 | 0.0566 | + | + | ++ |
| SRPX2 | 0.01 | 0.0194 | + | + | ++ |
| THOP1 | 0.01 | 0.0346 | + | + | ++ |

**NA,** no significant association with overall or disease free survival (p >0.05)

**Table 5: Markers related with HER2 breast cancer. The symbols "+", "-", "+/-", and "++" indicate the suitability of the markers for the determinations.**

| | | | **preferred** | **preferred** | **preferred** |
|---|---|---|---|---|---|
| | **OS** | **DFS** | **OS** | **DFS** | **OS/DFS** |
| AP1G1 | 0.002 | 0.0006 | + | + | ++ |
| BACH1 | NA | 0.0191 | - | + | -/+ |
| CDK2 | 0.0122 | NA | + | - | +/- |
| CFDP1 | NA | 0.0155 | - | + | -/+ |
| CHD2 | 0.032 | NA | + | - | +/- |
| DTL | NA | 0.0019 | - | + | -/+ |
| DYNC1H1 | 0.02 | NA | + | - | +/- |
| FN1 | 0.013 | 0.0143 | + | + | ++ |
| HPRT1 | NA | 0.0106 | - | + | -/+ |
| IRX5 | 0.026 | 0.0314 | + | + | ++ |
| KRT18 | 0.018 | 0.0074 | + | + | ++ |
| LMNB1 | NA | 0.0005 | - | + | -/+ |
| NRP2 | 0.02 | NA | + | - | +/- |
| OGFOD1 | 0.03 | NA | + | - | +/- |
| PSMD12 | NA | 0.0085 | - | + | -/+ |
| RAB3D | 0.02 | NA | + | - | +/- |
| SETD8 | 0.0028 | 0.0563 | + | + | ++ |
| SOCS3 | 0.0091 | NA | + | - | +/- |
| SRPK1 | NA | 0.0018 | - | + | -/+ |
| TMEM132A | 0.0026 | NA | + | - | +/- |
| TUBA1C | 0.031 | NA | + | - | +/- |
| TUBB2C | 0.0031 | NA | + | - | +/- |
| TUBB3 | 0.0004 | 0.0595 | + | + | ++ |
| ZC3H12A | 0.036 | NA | + | - | +/- |
| ZWINT | NA | 0.0041 | - | + | -/+ |

**NA,** no significant association with overall or disease free survival (p >0.05)

Another aspect of the disclosure thus relates to panels of markers that fulfill certain statistical quality parameters, such as a p-value of 0.1 and less, preferably 0.01 and less, and most preferred of 0.001 and less. Preferred is a panel of biomarkers for predicting response or resistance to specific therapies (overall survival, OS) in a breast cancer patient, wherein said panel consists of at least two of said biomarkers as disclosed, wherein said panel has an overall p-value for overall survival (OS) in said patient of 0.01 and less.

Disclosed is a biomarker, wherein said (at least one) marker has a p-value for overall survival (OS) in said patient of 0.05 and less, and is selected from the group consisting of ABCC5, ABHD5, ACLY, ACOT7, ALG8, AP1G1, APEX2, ARG2, ASPM, ATAD2, ATP9A, BLM, BOP1, BRCA1, CCNE1, CCNE2, CCNF, CCT5, CDC45L, CDC7, CDK2, CENPA, CENPE, CENPF, CEP55, CFDP1, CHAC1, CHD2, CLN6, CNOT6, COL13A1, CPOX, CTNNG, CTPS, CTSA, DDEF2, DDX46, DHFR, DONSON, DTL, E2F1, E2F8, ECT2, EML4, ESPL1, FAM46A, FANCI, GALNT14, GCLM, GTSE1, HIP1R, HMGCR, HPRT1, HSPC171, HYPK, IFIT1, IFIT3, KIF11, KRT19, LARP4, LCN2, LMNB1, LSR, MCM10, MCM3, MCM6, MCM7, MLF1IP, MMP1, MORC2, MSH6, MT1X, NCAPG, NUP107, OGFOD1, P2RY2, PCNA, PDCD6IP, PDSS1, PGK1, PHGDH, PKMYT1, POLQ, PRC1, PSMD12, PUS1, RACGAP1, RBM14, RPGRIP1L, RRM2, SETD8, SEZ6L2, SLC25A13, SPC25, SQLE, SRPK1, STC1, TFB2M, THOP1, TK1, TMEM132A, TMEM16A, TRIP13, TSEN2, TSPAN13, TTK, TUBA1B, TUBB2C, TUBB3, TXNRD1, ZC3H12A and ZWINT.

Disclosed is a biomarker, wherein said at least one marker has a p-value for overall survival (OS) in said patient of 0.01 and less, and is selected from the group consisting of ABCC5, ACOT7, AP1G1, APEX2, ARG2, ASPM, BRCA1, CCNE1, CCNE2, CCNF, CCT5, CD45L, CDC7, CENPA, CENPE, CENPF, CEP55, CFDP1, COL13A1, CPOX, CTNNG, CTPS, DDEF2, DONSON, DTL, E2F1, E2F8, ESPL1, GCLM, GTSE1, HMGCR, HPRT1, HPSC171, IFIT1, IFIT3, KIF11, LCN2, LMNB1, LSR, MCM10, MCM3, MCM6, MCM7, MLF1IP, MMP1, MORC2, MSH6, NCAPG, OGFOD1, PKMYT1, POLQ, PRC1, PSMD12, RACGAP1, RRM2, SETD8, SEZ6L2, SPC25, SQLE, SRPK1, STC1, THOP1, TK1, TMEM132A, TRIP13, TUBB2C, TUBB3, TXNRD1, ZWINT.

Disclosed is a biomarker, wherein said at least one marker has both a p-value for overall survival (OS) in said patient of 0.05 and less and a p-value for disease free survival (DFS) in said patient of 0.05 and less, and is selected from the group consisting of ACOT7, AP1G1, ASPM, ATAD2, ATP9A, BOP1, BRCA1, CCNE1, CCNE2, CCNF, CCT5, CDC45L, CDC7, CENPA, CENPE, CENPF, CEP55, CFDP1, CHAC1, CNOT6, COL13A1, DDEF2, DDX46, DHFR, DONSON, DTL, E2F1, E2F8, ECT2, ESPL1, EZH2, FANCI, GALNT14, GCLM, GTSE1, HMGCR, HPRT1, HPSC171, HYPK, IFIT1, IFIT3, KIF11, KRT19, LARP4, LMNB1, LSR, MCM10, MCM3, MCM6, MCM7, MLF1IP, MMP1, MORC2, MSH6, MT1X, NCAPG, NUP107, PCNA, PDSS1, PGK1, PHGDH, PRC1, PSMD12, RACGAP1, RPGRIP1L, RRM2, SETD8, SLC25A13, SPC25, SQLE, SRPK1, TFB2M, THOP1, TK1, TMEM132A, TRIP13, TSPAN13, TTK, TUBB2C, TUBB3, TXNRD1.

Further preferred are groups ("panels") of markers that are selected from the above markers. These panels can be, for example, biomarkers that can be used to identify risk groups, for example, groups of low or high risk. The low risk group could be spared of therapy, and the high risk groups would be chosen to receive certain therapies e.g. chemotherapy or others. This issue is of particular importance in node-negative patients. Node-negative means no detectable metastasis in locoregional lymph nodes (for example the sentinel lymph node). This group of patients does not need any chemotherapy, nevertheless, there can be 30% of high risk patients in this group, and highly significant DFS markers could identify these patients. The classification of tumors with highly significant OS biomarkers may aid to design more aggressive or specific therapies which are able to overcome the resistant state of such tumors, or can be used to design strategies that prevent the development of chemotherapy resistance (as well).

Preferred is the method according to the present invention, wherein said breast cancer is chemotherapy-resistant or will develop resistance to therapy, and progressing or relapsing breast cancer, as described above.

Further disclosed is a method, wherein a panel of biomarkers is used having an overall p-value for overall survival (OS) in said patient of 0.01 and less and/or a p-value for disease free survival (DFS) in said patient of 0.01 and less, preferably of 0.001 and less.

Based on the analysis of the biomarkers as identified following a patient-unspecific clinical treatment scheme (see above), another aspect of the method as disclosed then relates to a step of selecting patient- and/or cancer-specific biomarkers, and thus generating of a profile (or panel) of markers, that are of particular relevance for the individual patient and/or cancer, or for a (sub)group of patients (cohort) of certain patients. Thus, disclosed is a method, further comprising the step of generating an expression profile and/or activity profile based on said biomarkers, wherein said profile consists of biomarkers that are indicative for the overall survival and/or the disease-free survival of said cancer patient, and/or are cancer and/or patient specific. This selection of markers also has the advantage that not all markers have to be analyzed all the time, without that the (statistical and/or clinical) relevance of the analysis regarding the cancer and/or patient is insufficient.

In the methods of the present invention, in general the biomarkers can be detected and/or determined using any suitable assay. Detection is usually directed at the qualitative information ("marker yes-no"), whereas determining involves analysis of the quantity of a marker (e.g. expression level and/or activity). Detection is also directed at identifying mutations that cause altered functions of individual markers. The choice of the assay(s) depends on the parameter of the marker to be determined and/or the detection process. Thus, the determining and/or detecting can preferably comprise a method selected from subtractive hybridization, microarray analysis, DNA sequencing, qPCR, ELISA, enzymatic activity tests, cell viability assays, for example an MTT assay, phosphoreceptor tyrosine kinase assays, phospho-MAPK arrays and proliferation assays, for example the BrdU assay, proteomics, and mass spectrometry.

Preferably, said method is also amenable to automatization, and said activity and/or expression is preferably assessed in an automated and/or high-throughput format. Usually, this involves the use of chips and respective machinery, such as robots.

In the context of the present invention, any biological sample obtained from said cancer patient can be used, as long as it contains (or is presumed to contain) at least one of the biomarker(s) to be used in the analysis. Preferably, said sample is selected from tumor tissue (preferably breast tumor or metastases), biopsies, whole blood, peripheral blood, or fractions thereof, serum, buffy coat, lymphatic fluid, urine, bone marrow, EDTA plasma, heparinized plasma, citrate plasma, heparinized whole blood, and frozen samples thereof, such as frozen heparinized whole blood.

Disclosed is a method for treating and/or preventing breast cancer, in particular chemotherapy-resistance in a patient in need of said treatment, comprising the steps of a) performing a method according to the present invention a above, and b) providing a suitable chemotherapeutic anti-cancer treatment to said patient, wherein said treatment is based, at least in part, on the results of the method according to the present invention. Optionally, steps a) and b) of the method can be repeated.

"Treatment" shall mean a reduction and/or amelioration of the symptoms of the disease, here breast cancer. An effective treatment achieves, for example, a shrinking of the mass of a tumor and the number of cancer cells. A treatment can also avoid (prevent) and reduce the spread of the cancer, such as, for example, affect metastases and/or the formation thereof. A treatment may be a naïve treatment (before any other treatment of the cancer had started), or a treatment after the first round of treatment (e.g. after surgery or after a relapse or following the development of resistance, such as, for example, multidrug-resistant (MDR) cancer). The treatment can also be a combined treatment, involving, for example, chemotherapy, surgery, and/or radiation treatment.

Disclosed is a method, further comprising selecting a cancer-specific and/or patient-specific therapy for the treatment of breast cancer based on the results of the method according to the invention, preferably by improving the clinical endpoints overall survival. In practice, this is done by providing a treatment and/or treatment scheme for the patient that has a positive effect on the analysis as performed based on the biomarker(s) of the invention, in particular on the patient-specific panel of markers as identified according to the above. A positive effect can be assumed once the expression and/or activity of the markers as identified as being relevant for the patients' OS is modified, for example in that the over-expression of one or more of the markers is reduced.

Disclosed is a method, wherein said method also comprises administering to said patient an anti-cancer agent as identified with a method according to the invention as above.

Disclosed is a method, wherein said method also comprises monitoring said treatment of said patient as above, comprising repeating said method according to the invention as above during the treatment. A positive effect can be assumed once the expression and/or activity of the markers as identified as being relevant for the patients' OS is modified, for example in that the over-expression of one or more of the markers is reduced.

Disclosed is a method of predicting and/or determining the progression of a breast cancer, comprising the identification of at least one biomarker according to the present invention in an untreated tumor, and predicting response or resistance to specific therapies (overall survival, OS) and/or predicting the prognosis (relapse or progression) of the tumor in said breast cancer patient (disease free survival, DFS), based on said at least one biomarker as identified in said untreated tumor. In one preferred embodiment, said progression involves the development of chemotherapy resistance in said tumor.

Disclosed is a diagnostic kit comprising materials for performing a method according to the present invention in one or separate containers, preferably comprising at least one biomarker-specific antibody, optionally together with auxiliary agents and/or instructions for performing said method.

Disclosed are diagnostic kits ("panel kits") relating to specific marker combinations that are selected from the above markers. These panels can be, for example, biomarkers that can be used to identify risk groups, for example, groups of low or high risk. The kit would be used to identify a low risk group that would be spared of therapy, and a high risk group would be chosen to receive certain therapies e.g chemotherapy or others. This issue is of particular importance in node-negative patients. Node-negative means no detectable metastasis in locoregional lymph nodes (for example the sentinel lymph node). This group of patients does not need any chemotherapy, nevertheless, there can be 30% of high risk patients in this group, and highly significant DFS markers could identify these patients. Furthermore, the classification of tumors with highly significant OS biomarkers may aid to design more aggressive or specific therapies which are able to overcome the resistant state of such tumors, or can be used to design strategies that prevent the development of chemotherapy resistance (as well).

It is very important to note that the KM plotter database for breast cancer, as used in the present invention, contains microarray data from breast cancer patients whose tumours were obtained during surgery. These tumours are treatment naive, i.e. have not yet been treated by any therapy (chemotherapy, radiation, targeted therapy). By using the present approach as described, the inventors were able to identify genes that are already expressed in breast cancer tissue at the time of surgery (i.e. before treatment/untreated). The KM plotter analysis shows which genes of the resistance signature are present in the untreated tumours, and the analysis unveils which of these genes determine prognosis (DFS, disease free survival), and which of these genes (overall survival, OS) are associated with response or resistance to therapy.

The invention shall now be further described in the following examples with reference to the accompanying Figures, nevertheless, without being limited thereto. In the Figures,

Figure 1 as an example shows the development of cisplatin resistance during a treatment with weekly doses of cisplatin as used in the clinic.

Figure 2 as an example shows the Kaplan-Meier analysis of SQLE gene expression in breast cancer patients.

Table 6 shows the official gene symbols, Affymetrix ID numbers and the Agilent ID numbers for the biomarkers as disclosed

### Examples

### Identification of novel prognostic and predictive breast cancer biomarkers

### 1. Definition and background (Hayes et al. (1996).

Tumor marker utility grading system: a framework to evaluate clinical utility of tumor markers. JNCI 88, 1456-1466)

Prognosis for patients with established primary or metastatic cancer is defined as the prediction of future behavior of an established malignancy, either in the absence of or after application of therapy (local or systemic). Prognostic factors are divided into two fundamentally different categories: 1) those that predict relapse or progression independent of future treatment effects (which are designated "prognostic" factors) and 2) those that predict response or resistance to a specific therapy (which are designated "predictive" factors). So far, only very few biomarkers for breast cancer have demonstrated clinical utility. For the identification of novel prognostic and predictive biomarkers it was therefore necessary to develop an efficient method which fulfils this task. This is especially important as relapsed or therapy resistant breast cancer tissue is normally not available also for ethical reasons (discussed by Sawyers CL (2008). The cancer biomarker problem. Nature 425, 548-552).

### 2. Identification of gene panels (signatures) for breast cancer

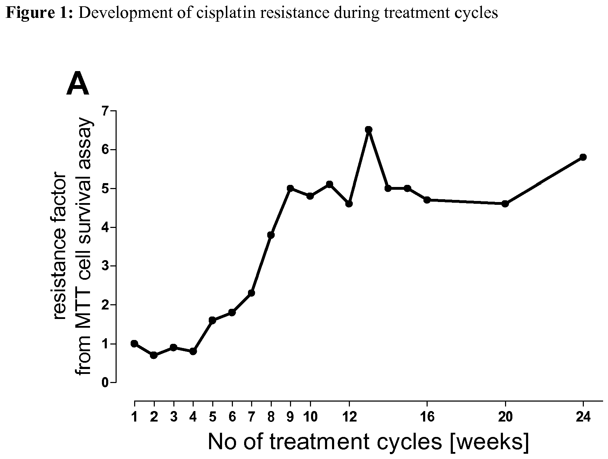

For the identification of novel prognostic and predictive breast cancer biomarkers, cisplatin resistant breast cancer cell lines were established following protocols that were developed based on Eckstein et al. and Gosepath et al. (Eckstein N, Servan K, Girard L, Cai D, von Jonquieres G, Jaehde U, Kassack MU, Gazdar AF, Minna JD, Royer HD. Epidermal growth factor receptor pathway analysis identifies amphiregulin as a key factor for cisplatin resistance of human breast cancer cells. J Biol Chem. 2008 Jan 11; 283(2):739-50.; Gosepath EM, Eckstein N, Hamacher A, Servan K, von Jonquieres G, Lage H, Györffy B, Royer HD, Kassack MU. Acquired cisplatin resistance in the head-neck cancer cell line Ca127 is associated with decreased DKK1 expression and can partially be reversed by overexpression of DKK1. Int J Cancer. 2008 Nov 1;123(9):2013-9), see also Figure 1. In a second step, global gene expression levels were analyzed using dual color Agilent microarrays, and gene expression signatures for cisplatin resistance were established. Cisplatin was used as a representative genotoxic drug, which can be selected from DNA-damaging agents (genotoxic drugs) that are in use for breast cancer therapy, such as cyclophosphamide, carboplatin, cisplatin, doxorubicin, epirubicin, mitomycin C, radiation (radiotherapy), and PARP inhibitors, or busulfan, bendamustine, carboplatin, carmustine, chlorambuci, cyclophosphamide, dacarbazine, daunorubicin, etoposide, idarubicin, ifosfamide, irinotecan, lomustine, mechlorethamine, melphalan, mitoxantrone, oxaliplatin, temozolamide, and topotecan. This approach can be done similarly for any other chemotherapeutic drug and any other tumor. As an example, this approach was used similarly for ovarian cancer (Eckstein et al. (2009). Hyperactivation of the insulin-like growth factor receptor I signaling pathway is an essential event for cisplatin resistance of ovarian cancer cells. Cancer Res 69, 2996-3003) or head-neck cancer (Gosepath EM, Eckstein N, Hamacher A, Servan K, von Jonquieres G, Lage H, Györffy B, Royer HD, Kassack MU. Acquired cisplatin resistance in the head-neck cancer cell line Ca127 is associated with decreased DKK1 expression and can partially be reversed by overexpression of DKK1. Int J Cancer. 2008 Nov 1;123(9):2013-9). The resulting signatures typically contain 800 to 1600 genes, and evidently these genes (biomarkers) play a role for chemotherapy resistance.

### 3. KM Plotter "breast cancer"

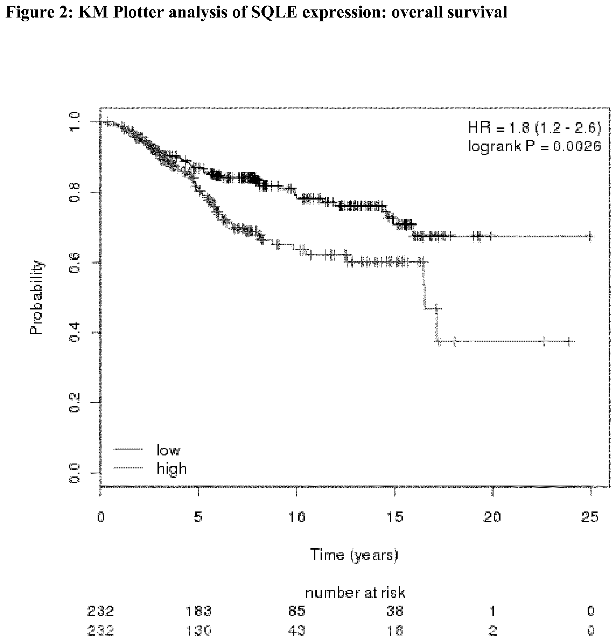

In order to identify prognostic and predictive biomarkers the inventors used a data base that contains the microarray expression data of 2472 breast cancer patients (kmplot.com). This database also contains the clinical data of these patients and it is possible to extract data on overall survival (OS) and disease free survival (DFS) of these patients. The KM Plotter breast cancer is able to draw Kaplan-Meier survival curves of the patient cohort for individual genes. For example, the Kaplan-Meier plot shows overall survival data of breast cancer patients and SQLE gene expression, the associated p value is 0.0026 (see Figure 2). The light gray curve represents patients with high SQLE expression and the black curve those with low SQLE expression. The expression levels of each gene in the database have been investigated and the appropriate values for high and low expression levels in the patients have been set.

To further identify the breast cancer biomarkers as present, this KM plotter breast cancer was employed. Each gene of a given "resistance" signature was manually entered into the KM plotter, and a Kaplan-Meier curve for survival is generated. For each gene, the inventors thus determined the overall survival (OS) and disease free survival (DFS) in the breast cancer cohort of the database.

Predictive biomarkers were identified by overall survival and prognostic biomarkers by disease-free survival analysis. Overall survival analysis is a gold standard for measuring the efficacy of a therapeutic regimen (or a combination of treatments) in cancer patients. Disease free survival is a prognostic parameter that addresses future tumor behavior in a patient.

### 4. Alternative risk assessment based on Gillet et al.

For each patient sample, a low/high prediction of risk can be calculated using either: a) the clinical covariates alone or b) biomarker gene expression alone or c) clinical covariates along with biomarker gene expression.

The clinical covariates used can be age, stage, and residual tumor (optimal vs. sub-optimal debulking).

Each method results in a log-rank statistic and the statistics for the two methods can be compared with each other to decide whether there is a statistically significant difference between the two. A statistically significant difference implies that the biomarker gene expression data add significantly to the risk prediction accuracy. The calculation of statistical significance for the difference was done using BRB ArrayTools by randomly shuffling the survival times to estimate the null distribution of the log-rank differences. A p-value for the observed difference was calculated using this null distribution.

Also used a two-step method to divide the subjects into four risk groups can be used. In the first step, the clinical covariates alone were used to divide the subjects into low and high risk groups. Then within each risk group, the members were further divided into two risk subgroups (denoted low/high) using the sum of the gene expressions weighted by their CPH coefficient. The median of this sum was used as a threshold to further subdivide each group into high and low. This gives a four-level risk prediction consisting of high/high, high/low, low/high and low/low groups.

**Table 6: Affymetrix* and Agilent ID numbers for all biomarkers as disclosed**

| **Marker** | **Affy ID** | **Agilent ID** | **Marker** | **Affy ID** | **Agilent ID** |
|---|---|---|---|---|---|
| ABCC5 | 209380_s_at | A_23_P258221 | ACOT7 | 208002_s_at | A_24_P205589 |
| ACCN2 | 205156_s_at | A_23_P204751 | ALG8 | 203545_at | A_23_P13554 |
| ACLY | 201128_s_at | A_23_P66787 | AP2A2 | 212161_at | A_24_P932418 |
| ALDH3B2 | 204942_s_at | A_23_P24311 | ASGR1 | 206743_s_at | A_23_P118722 |
| APEX2 | 204408_at | A_23_P256682 | CNOT6 | 217970_s_at | A_23_P167417 |
| ARG2 | 203945_at | A_23_P128728 | COL13A1 | 211343_s_at | A_23_P1331 |
| ASPM | 219918_s_at | A_23_P52017 | CSTF3 | 203947_at | A_23_P98310 |
| ATAD2 | 218782_s_at | A_24_P59596 | DDEF2 | 206414_s_at | A_24_P362540 |
| ATP9A | 212062_at | A_24_P135992 | DHFR | 202532_s_at | A_24_P320284 |
| BLM | 205733_at | A_23_P88630 | DDX46 | 202462_s_at | A_23_P30377 |
| BOP1 | 212563_at | A_23_P43800 | DHODH | 213632_at | A_23_P15202 |
| BRCA1 | 204531_s_at | A_23_P207400 | DLGAP4 | 202572_s_at | A_23_P210419 |
| CCNE1 | 213523_at | A_23_P209200 | DTL | 218585_s_at | A_23_P10385 |
| CCNE2 | 205034_at | A_23_P215976 | EAF1 | 212275_s_at | A_24_P941930 |
| CCNF | 204826_at | A_23_P37954 | GAA | 202812_at | A_23_P153026 |
| CCT5 | 208696_at | A_23_P257863 | GDI1 | 201864_at | A_23_P45496 |
| CDC25A | 204695_at | A_24_P397107 | HIP1R | 209558_s_at | A_23_P398294 |
| CDC45L | 204126_s_at | A_23_P57379 | IFIT1 | 203153_at | A_23_P52266 |
| CDC7 | 204510_at | A_23_P148807 | IRX5 | 210239_at | A_23_P9779 |
| CDK2 | 204252_at | A_23_P98898 | KRT18 | 201596_x_at | A_32_P151544 |
| CENPA | 204962_s_at | A_24_P413884 | KRT19 | 201650_at | A_23_P66798 |
| CENPE | 205046_at | A_23_P253524 | LIN7B | 219760_at | A_23_P164912 |
| CENPF | 209172_s_at | A_23_P401 | LSR | 208190_s_at | A_23_P142389 |
| CEP55 | 218542_at | A_23_P115872 | NEBL | 203961_at | A_23_P104522 |
| CFDP1 | 203166_at | A_23_P89123 | NSUN6 | 222128_at | A_23_P61580 |
| CHAC1 | 219270_at | A_23_P14863 | PDCD6IP | 217746_s_at | A_23_P60816 |
| CLN6 | 218161_s_at | A_23_P117797 | PGK1 | 200738_s_at | A_23_P125829 |
| CPOX | 204172_at | A_23_P144179 | PKP3 | 209873_s_at | A_23_P95810 |
| CTNNG | 201015_s_at | A_23_P501822 | PPFIA4 | 214978_s_at | A_23_P420692 |
| CTPS | 202613_at | A_23_P21706 | RACGAP1 | 222077_s_at | A_23_P65110 |
| CTSA | 200661 at | A_24_P74371 | RBM14 | 204178_s_at | A_23_P150255 |
| CTSL1 | 202087_s_at | A_23_P94533 | RFNG | 212968_at | A_23_P84629 |
| DONSON | 221677_s_at | A_23_P500390 | RUVBL1 | 201614_s_at | A_24_P148811 |
| E2F1 | 204947_at | A_23_P80032 | SEZ6L2 | 218720_x_at | A_23_P10194 |
| E2F8 | 219990 at | A_23_P35871 | SOD1 | 200642_at | A_23_P154840 |
| ECT2 | 219787_s_at | A_23_P9574 | STC1 | 204597_x_at | A_23_P314755 |
| EML4 | 220386_s_at | A_23_P143127 | TCOF1 | 202385_s_at | A_23_P310317 |
| EPN3 | 220318_at | A_23_P130027 | TFB2M | 218605_at | A_23_P45940 |
| ESPL1 | 38158_at | A_23_P32707 | TSEN2 | 219581_at | A_23_P92012 |
| EZH2 | 203358_s_at | A_23_P259641 | TSPAN13 | 217979_at | A_23_P168610 |
| FAM46A | 221766_s_at | A_23_P70660 | USP14 | 201671_x_at | A_24_P313744 |
| FANCI | 213007_at | A_23_P375104 | USP32 | 211702_s_at | A_23_P431630 |
| FREQ | 218266_s_at | A_23_P217049 | WDR26 | 218107_at | A_23_P85604 |
| GALNT14 | 219271_at | A_23_P67847 | ZC3H12A | 218810_at | A_23_P326160 |
| GARS | 208693_s_at | A_24_P154948 | ABHD5 | 213935_at | A_23_P250294 |
| GCLM | 203925_at | A_23_P103996 | AP1G1 | 203350_at | A_24_P381962 |
| GPNMB | 201141_at | A_23_P134426 | CD2AP | 203593_at | A_32_P155811 |
| GPR157 | 220901_at | A_23_P57643 | DMBT1 | 208250_s_at | A_23_P86599 |
| GTSE1 | 204318_s_at | A_24_P916195 | DYNC1H1 | 211928_at | A_23_P128706 |
| HMGCR | 202540_s_at | A_23_P30495 | HIP1R | 209558_s_at | A_23_P398294 |
| HMGCS1 | 221750_at | A_24_P63522 | HYPK | 218680_x_at | A_23_P117683 |
| HN1 | 217755_at | A_23_P100632 | LARP4 | 212714_at | A_24_P222365 |
| HPRT1 | 202854_at | A_23_P11372 | LCN2 | 212531_at | A_23_P169437 |
| IFIT3 | 204747_at | A_23_P35412 | MMP1 | 204475_at | A_23_P1691 |
| KIF11 | 204444_at | A_24_P227091 | MT1X | 204326_x_at | A_23_P303242 |
| KRT19 | 201650_at | A_23_P66798 | PDCD6IP | 217746_s_at | A_23_P60816 |
| LMNB1 | 203276_at | A_23_P258493 | RPGRIP1L | 213959_s_at | A_23_P388489 |
| MCM10 | 220651_s_at | A_24_P412088 | SLC25A13 | 203775_at | A_23_P82334 |
| MCM3 | 201555_at | A_23_P7873 | SRPX2 | 205499_at | A_23_P136978 |
| MCM7 | 208795_s_at | A_23_P93690 | THOP1 | 203235_at | A_23_P28057 |
| MCM6 | 201930_at | A_23_P90612 | TSEN2 | 219581_at | A_23_P92012 |
| MLF1IP | 218883_s_at | A_23_P254733 | AP1G1 | 203350_at | A_24_P381962 |
| MORC2 | 203956_at | A_24_P100234 | ARID3A | 205865_at | A_23_P16516 |
| MSH6 | 202911_at | A_23_P102202 | BACH1 | 204194_at | A_23_P211047 |
| MYOHD1 | 219320_at | A_23_P100868 | CEACAM6 | 211657_at | A_23_P218442 |
| NCAPG | 218662_s_at | A_23_P155815 | CHD2 | 203461_at | A_24_P264664 |
| NEIL3 | 219502_at | A_23_P155711 | DYNC1H1 | 211928_at | A_23_P128706 |
| NFKBIL2 | 207137_at | A_23_P216355 | FA2H | 219429_at | A_23_P49448 |
| NUDT4 | 212183_at | A_24_P335263 | FN1 | 212464_s_at | A_24_P119745 |
| NUP107 | 218768_at | A_23_P14062 | HSPC171 | 221597_s_at | A_23_P206369 |
| P2RY2 | 206277_at | A_23_P24903 | IRX5 | 210239_at | A_23_P9779 |
| PCNA | 201202_at | A_23_P28886 | KRT18 | 201596_x_at | A_23_P99320 |
| PDSS1 | 220865_s_at | A_23_P161152 | LMNB1 | 203276_at | A_23_P258493 |
| PKMYT1 | 204267_x_at | A_24_P105102 | NRP2 | 203413_at | A_23_P429555 |
| POLQ | 219510_at | A_23_P218827 | NSUN6 | 222128_at | A_23_P61580 |
| PRC1 | 218009_s_at | A_23_P206059 | OGFOD1 | 221090_s_at | A_23_P3775 |
| PSMD12 | 202352_s_at | A_32_P12639 | PHGDH | 201397_at | A_23_P85783 |
| PUS1 | 218670_at | A_23_P251771 | RAB3D | 208466_at | A_23_P404678 |
| RACGAP1 | 222077_s_at | A_32_P186474 | RRM1 | 201477_s_at | A_23_P87351 |
| RRM2 | 201890_at | A_24_P234196 | SEC61A2 | 219499_at | A_23_P161197 |
| SETD8 | 220200_s_at | A_32_P191859 | SETD8 | 220200_s_at | A_32_P191859 |
| SOD1 | 200642_at | A_23_P154840 | SOCS3 | 206359_at | A_23_P207058 |
| SPC25 | 209891_at | A_23_P51085 | TMEM132A | 218834_s_at | A_23_P24716 |
| SRPK1 | 202200_s_at | A_23_P19543 | TUBA1C | 209251_x_at | A_23_P128154 |
| SQLE | 209218_at | A_23_P146284 | TUBB3 | 202154_x_at | A_24_P258633 |
| TK1 | 202338_at | A_23_P107421 | TUBA1B | 211058_x_at | A_23_P128147 |
| TMEM132A | 218834_s_at | A_23_P24716 | TUBB2C | 213726_x_at | A_32_P187327 |
| TMEM16A | 218804_at | A_23_P98304 | ZC3H12A | 218810_at | A_23_P326160 |
| TRIP 13 | 204033_at | A_24_P277576 | ZWINT | 204026_s_at | A_23_P63789 |
| TTK | 204822_at | A_23_P259586 | | | |
| TUBA1B | 211058_x_at | A_23_P128147 | | | |
| TUBB | 204141_at | A_32_P78528 | | | |
| TXNRD1 | 201266_at | A_23_P204581 | | | |
| UCHL5 | 220083_x_at | A_23_P148798 | | | |
| WRN | 205667_at | A_23_P250813 | | | |

| | | | | | |
|---|---|---|---|---|---|
| * The Affymetrix IDs represent the best fit oligonucleotide for a given gene when multiple Affy ID numbers are (oligonucleotides) are available on the microarray. These are listed in the KM plotter as JetSet probes (kmplot.com) | | | | | |

## Claims

1. A method for predicting response or resistance to specific therapies (overall survival, OS) in a breast cancer patient that is undergoing cancer treatment, comprising determining the expression level of the biomarker ASPM in a biological sample obtained from said cancer patient,
wherein an increase or higher level of expression of the biomarker ASPM compared to the median thereof in a given cancer patient population is predictive for an adverse response or resistance outcome for said patient.

2. The method according to claim 1, wherein said breast cancer is chemotherapy-resistant breast cancer, and/or luminal A breast cancer.

3. The method according to claim 1 or 2, wherein said marker has an overall p-value for overall survival (OS) in said patient of 0.05 and less.

4. The method according to any of claims 1 to 3, wherein said determining comprises a method selected from subtractive hybridization, microarray analysis, mutation analysis by DNA sequencing, qPCR, ELISA, enzymatic activity tests, cell viability assays, for example an MTT assay, phosphoreceptor tyrosine kinase assays, phospho-MAPK arrays and proliferation assays, for example the brdU assay, proteomics, and mass spectrometry.

5. The method according to any of claims 1 to 4, wherein furthermore the expression level and/or biological activity of at least one additional biomarker selected from the group of ABCC5, ABHD5, ACLY, ACOT7, ALG8, AP1G1, APEX2, ARG2, ATAD2, ATP9A, BACH1, BLM, BOP1, BRCA1, CCNE1, CCNE2, CCNF, CCT5, CD2AP, CDC25A, CDC45L, CDC7, CDK2, CENPA, CENPE, CENPF, CEP55, CFDP1, CHAC1, CHD2, CLN6, CNOT6, COL13A1, CPOX, CTNNG, CTPS, CTSA, CTSL1, DDEF2, DDX46, DHFR, DONSON, DTL, DYNC1H1, E2F1, E2F8, ECT2, EML4, ESPL1, EZH2, FA2H, FAM46A, FANCI, FN1, FREQ, GALNT14, GARS, GCLM, GPNMB, GTSE1, HIP1R, HMGCR, HMGCS1, HN1, HPRT1, HSPC171, HYPK, IFIT1, IFIT3, KIF11, KRT19, LARP4, LCN2, LMNB1, LSR, MCM10, MCM3, MCM6, MCM7, MLF1IP, MMP1, MORC2, MSH6, MT1X, NCAPG, NUDT4, NUP107, OGFOD1, P2RY2, PCNA, PDCD6IP, PDSS1, PGK1, PHGDH, PKMYT1, POLQ, PPFIA4, PRC1, PSMD12, PUS1, RAB3D, RACGAP1, RBM14, RPGRIP1L, RRM1, RRM2, RUVBL1, SETD8, SEZ6L2, SLC25A13, SOD1, SPC25, SQLE, SRPK1, STC1, TFB2M, THOP1, TK1, TMEM132A, TMEM16A, TRIP13, TSEN2, TSPAN13, TTK, TUBA1B, TUBB, TUBB2C, TUBB3, TXNRD1, UCHL5, USP32, WDR26, and ZWINT is determined.

## Patentansprüche

1. Verfahren zur Vorhersage der Antwort oder Resistenz auf spezifische Therapien (Gesamt-Überleben, OS) in einer Brustkrebspatientin, die eine Krebsbehandlung unterzogen wird, umfassend
Bestimmen des Expressionsspiegels des Biomarker ASPM in einer biologischen Probe erhalten von dem Krebspatienten,
wobei eine Erhöhung oder höherer Spiegel der Expression des Biomarkers ASPM verglichen zu dem Median davon in einer bestimmten Krebspatientenpopulation für eine nachteilige Antwort oder Resistenzausgang für den Patienten prädiktiv ist.

2. Verfahren nach Anspruch 1, wobei der Brustkrebs Chemotherapie-resistenter Brustkrebs und/oder Luminal A Brustkrebs ist.

3. Verfahren nach Anspruch 1 oder 2, wobei der Marker einen Gesamt p-Wert für Gesamt-Überleben (OS) in der Patientin von 0,05 und weniger aufweist.

4. Verfahren nach einem der Ansprüche 1 bis 3, wobei das Bestimmen ein Verfahren ausgewählt aus subtraktiver Hybridisierung, Mikroarrayanalyse, Mutationsanalyse durch DNA Sequenzierung, qPCR, ELISA, enzymatische Aktivitätstests, Zell-Lebensfähigkeitstest, zum Beispiel einen MTT Test, Phosphorezeptor Tyrosinkinasetests, Phospho-MAPK-Tests und Zellteilungstests, zum Beispiel den brdU Test, Proteomics und Massenspektrometrie umfasst.

5. Verfahren nach einem der Ansprüche 1 bis 4, wobei weiterhin der Expressionsspiegel und/oder die biologische Aktivität von mindestens einem zusätzlichen Biomarker ausgewählt aus der Gruppe von ABCC5, ABHD5, ACLY, ACOT7, ALG8, AP1G1, APEX2, ARG2, ATAD2, ATP9A, BACH1, BLM, BOP1, BRCA1, CCNE1, CCNE2, CCNF, CCT5, CD2AP, CDC25A, CDC45L, CDC7, CDK2, CENPA, CENPE, CENPF, CEP55, CFDP1, CHAC1, CHD2, CLN6, CNOT6, COL13A1, CPOX, CTNNG, CTPS, CTSA, CTSL1, DDEF2, DDX46, DHFR, DONSON, DTL, DYNC1H1, E2F1, E2F8, ECT2, EML4, ESPL1, EZH2, FA2H, FAM46A, FANCI, FN1, FREQ, GALNT14, GARS, GCLM, GPNMB, GTSE1, HIP1R, HMGCR, HMGCS1, HN1, HPRT1, HSPC171, HYPK, IFIT1, IFIT3, KIF11, KRT19, LARP4, LCN2, LMNB1, LSR, MCM10, MCM3, MCM6, MCM7, MLF1IP, MMP1, MORC2, MSH6, MT1X, NCAPG, NUDT4, NUP107, OGFOD1, P2RY2, PCNA, PDCD6IP, PDSS1, PGK1, PHGDH, PKMYT1, POLQ, PPFIA4, PRC1, PSMD12, PUS1, RAB3D, RACGAP1, RBM14, RPGRIP1L, RRM1, RRM2, RUVBL1, SETD8, SEZ6L2, SLC25A13, SOD1, SPC25, SQLE, SRPK1, STC1, TFB2M, THOP1, TK1, TMEM132A, TMEM16A, TRIP13, TSEN2, TSPAN13, TTK, TUBA1B, TUBB, TUBB2C, TUBB3, TXNRD1, UCHL5, USP32, WDR26 und ZWINT bestimmt wird.

## Revendications

1. Méthode permettant de prédire la réponse ou la résistance à des thérapies spécifiques (survie globale, OS) chez une patiente atteinte de cancer du sein sous traitement anticancéreux, comprenant la détermination du niveau d'expression du biomarqueur ASPM dans un échantillon biologique provenant de ladite patiente atteinte de cancer,
dans laquelle un accroissement ou un niveau plus élevé du biomarqueur ASPM comparativement à sa valeur médiane chez une population donnée de patientes atteintes de cancer prédit un résultat négatif ou une résistance chez ladite patiente.

2. Méthode selon la revendication 1, dans laquelle ledit cancer du sein est un cancer du sein résistant à la chimiothérapie et/ou un cancer du sein de type luminal A.

3. Méthode selon la revendication 1 ou 2, dans laquelle ledit marqueur a une valeur p globale en termes de survie globale (OS) chez ladite patiente de 0,05 et moins.

4. Méthode selon l'une quelconque des revendications 1 à 3, dans laquelle ladite détermination comprend une méthode choisie parmi l'hybridation soustractive, l'analyse sur micropuces, l'analyse des mutations par séquençage ADN, la PCRq, ELISA, les tests d'activité enzymatique, les dosages de viabilité cellulaire, par exemple un dosage MTT, les dosages de tyrosine kinase des phosphorécepteurs, les puces à phospho-MAPK et les dosages de prolifération, par exemple le dosage brdU, la protéomique, et la spectrométrie de masse.

5. Méthode selon l'une quelconque des revendications 1 à 4, dans laquelle en plus le niveau d'expression et/ou l'activité biologique d'au moins un biomarqueur supplémentaire choisi dans le groupe ABCC5, ABHD5, ACLY, ACOT7, ALG8, AP1G1, APEX2, ARG2, ATAD2, ATP9A, BACH1, BLM, BOP1, BRCA1, CCNE1, CCNE2, CCNF, CCT5, CD2AP, CDC25A, CDC45L, CDC7, CDK2, CENPA, CENPE, CENPF, CEP55, CFDP1, CHAC1, CHD2, CLN6, CNOT6, COL13A1, CPOX, CTNNG, CTPS, CTSA, CTSL1, DDEF2, DDX46, DHFR, DONSON, DTL, DYNC1H1, E2F1, E2F8, ECT2, EML4, ESPL1, EZH2, FA2H, FAM46A, FANC1, FN1, FREQ, GALNT14, GARS, GGLM, GPNMB, GTSE1, HIP1R, HMGCR, HMGCS1, HN1, HPRT1, HSPC171, HYPK, IFIT1, IFIT3, KIF11, KRT19, LARP4, LCN2, LMNB1, LSR, MCM10, MCM3, MCM6, MCM7, MLF1IP, MMP1, MORC2, MSH6, MT1X, NCAPG, NUDT4, NUP107, OGFOD1, P2RY2, PCNA, PDCD6IP, PDSS1, PGK1, PHGDH, PKMYT1, POLQ, PPFIA4, PRC1, PSDM12, PUS1, RAB3D, RACGAP1, RBM14, RPGRIP1L, RRM1, RRM2, RUVBL1, SETD8, SEZ6L2, SLC25A13, SOD1, SPC25, SQLE, SRPK1, STC1, TFB2M, THOP1, TK1, TMEM132A, TMEM16A, TRIP13, TSEN2, TSPAN13, TTK, TUBA1B, TUBB, TUBB2C, TUBB3, TXNRD1, UCHL5, USP32, WDR26, et ZWINT est déterminé.
